# EUROPEAN PATENT SPECIFICATION

(11) **EP 4 380 516 B1**
(45) Date of publication and mention of the grant of the patent: **02.12.2026**
(21) Application number: 22757780.6
(22) Date of filing: 01.08.2022
(51) Int. Cl.: A61F 5/451, A61F 5/455

(54) **EXTRUDED FLUID COLLECTION DEVICES**
EXTRUDIERTE FLÜSSIGKEITSSAMMELVORRICHTUNGEN
DISPOSITIFS DE COLLECTE DE FLUIDE EXTRUDÉS

(30) Priority: 02.08.2021 US 202163228252 P
(43) Date of publication of application: 12.06.2024
(73) Proprietor: Purewick Corporation, El Cajon, California 92020 (US)
(72) Inventor: JULIAN, Alexandra, Elmhurst, Illinois 60126 (US); RANALLETTA, Joseph Vincent, Greenville, South Carolina 29601 (US); WANG, Gonghao, Cary, North Carolina 30002 (US); HAYES, John, Raleigh, North Carolina 27613 (US)
(74) Representative: Hoffmann Eitle
(86) International application number: PCT/US2022/039018
(87) International publication number: WO 2023/014639

(56) References cited:
- WO-A1-2019/212949
- WO-A1-2019/212949
- WO-A1-2021/007144
- WO-A1-2021/016026
- WO-A1-2022/035745
- WO-A1-2022/035745
- WO-A1-2022/051360
- US-A1- 2001 037 097
- US-A1- 2016 374 848
- US-A1- 2016 374 848

## Description

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections.

Thus, users and manufacturers of fluid collection devices continue to seek new and improved devices, systems, and methods to collect urine.

### SUMMARY

The invention is defined in claim 1 below. The dependent claims are directed to optional features and preferred embodiments. Disclosed is a fluid collection device that includes a fluid impermeable barrier and an elongated body that is at least partially fluid permeable. The fluid impermeable barrier has a distal end region and a proximal end region defining an aperture sized and dimensioned to have urine drawn therethrough by a vacuum source. The elongated body has a portion of the body between the distal end region and the proximal end region that is free from being covered by the fluid impermeable barrier and positioned on the fluid collection device to receive urine discharged from a urethra at least proximate to the portion of the body. For disclosures of such devices see for example Applicant's WO2019/212949, WO2022/035745 and US2016/374848. The fluid collection device disclosed herein includes at least an extruded support.

Features from any of the embodiments of the invention disclosed below may be used in combination with one another, provided that the combination falls within the scope of the appended claims. In addition, other features and advantages of the present invention will become apparent to those of ordinary skill in the art through consideration of the following detailed description and the accompanying drawings.

### BRIEF DESCRIPTION OF THE DRAWINGS

In the drawings identical reference numerals refer to identical or similar elements or features in different views or embodiments shown in the drawings.
**FIG. 1** is a block diagram of a fluid collection system.
**FIG. 2A** is a front isometric view of a fluid collection device.
**FIG. 2B** is a longitudinal cross-sectional view of the fluid collection device of **FIG. 2A****.**
**FIG. 2C** is an exploded view of the fluid collection device of **FIG. 2A****.**
**FIG. 3A** is a front isometric view of a fluid collection device.
**FIG. 3B** is a longitudinal cross-sectional view of the fluid collection device of **FIG. 3A****.**
**FIG. 3C** is an exploded view of the fluid collection device of **FIG. 3A****.**
**FIG. 4** is an exploded view of a fluid collection device.
**FIG. 5A** is a front isometric view of a fluid collection device, according to an embodiment.
**FIG. 5B** is a transverse cross-sectional view of the fluid collection device of **FIG. 5A****.**
**FIG. 5C** is an exploded view of the fluid collection device of **FIG. 5A****.**
**FIG. 5D** is a longitudinal cross-sectional view of the fluid collection device of **FIG. 5A****.**
**FIG. 6A** is a front isometric view of a fluid collection device, according to an embodiment.
**FIG. 6B** is a transverse cross-sectional view of the fluid collection device of **FIG. 6A****.**
**FIG. 6C** is an exploded view of the fluid collection device of **FIG. 6A****.**
**FIG. 7** is a flowchart of a method of manufacturing a fluid collection device.
**FIG. 8** is a flowchart of a method of manufacturing a fluid collection device.

### DETAILED DESCRIPTION

The devices and systems disclosed herein are configured to collect fluids from an individual. The fluids collected by the fluid collection devices may include at least one of urine, vaginal discharge, penile discharge, reproductive fluids, blood, sweat, wound exudate, or other bodily fluids. Efficient and repeatable manufacturing and assembly of fluid collection devices result in the technical effect of repeatability and reducing the cost and/or time for assembly of the fluid collection device.

Non-claimed methods of manufacture and/or assembly may include one or more of: molding the exterior fluid impermeable barrier to receive the inner components (e.g., body and/or conduit) rather than using manpower to align the fluid impermeable barrier and the inner components; molding the fluid impermeable barrier with a flat back side such that only about half of the fluid permeable body is required in the fluid collection device; and/or extruding a three-layer structure that include an inner lumen (e.g., conduit), an extruded support, and a fluid permeable membrane in one structure that may be cut to length and capped at the ends (with one end having an aperture for the tubing).

Furthermore, embodiments of the fluid collection devices may include a shape memory material (*e.g*., bendable spine) that provides use benefits for positioning and securement for the user. Some embodiments may include incorporation of a label into the fluid collection device that includes instructions, product identification, and/or user-notated usage data. The incorporation of labeling in at least one, some, or all embodiments described herein results in the technical effect of reducing packaging required for the fluid collection device, providing information or instructions to the user in a place that is less likely to be discarded by the user, and/or providing a space to record relevant data pertaining to fluid collection of the user.

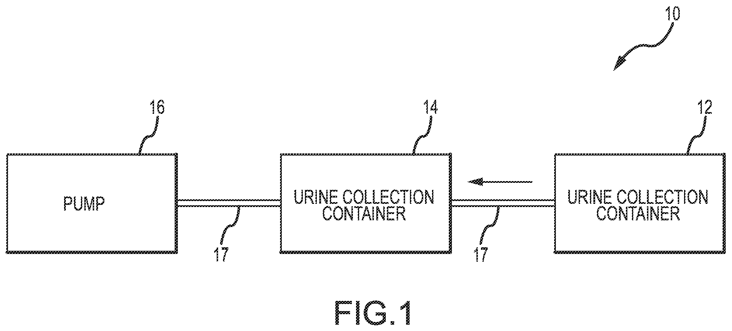

**FIG. 1** is a block diagram of a fluid collection system 10. The system 10 includes a fluid collection device 12 (*e.g.,* any of the fluid collection devices disclosed herein), a urine collection container 14, and a pump 16 (or vacuum source). The fluid collection device 12, the urine collection container 14, and the pump 16 may be fluidly coupled to each other via one or more conduits 17. For example, fluid collection device 12 may be operably coupled to one or more of the urine collection container 14 or the pump 16 via the conduit 17. The pump 16 may be secured directly to the urine collection container 14. Fluid (*e.g.,* urine or other bodily fluids) collected in the fluid collection device 12 may be removed from the fluid collection device 12 via the conduit 17 secured to the fluid collection device 12. Suction force may be introduced into the chamber of the fluid collection device 12 via the inlet of the conduit 17 responsive to suction (*e.g*., vacuum) force applied at the outlet of the conduit 17.

The suction force may be applied to the outlet of the conduit 17 by the pump 16 either directly or indirectly. The suction force may be applied indirectly via the urine collection container 14. For example, the outlet of the conduit 17 may be disposed within or fluidly coupled to an interior region of the urine collection container 14 and an additional conduit 17 may extend from the urine collection container 14 to the pump 16. Accordingly, the pump 16 may apply suction to the fluid collection device 12 via the urine collection container 14. The suction force may be applied directly via the pump 16. For example, the outlet of the conduit 17 may be disposed within the pump 16. An additional conduit 17 may extend from the pump 16 to a point outside of the fluid collection device 12, such as to the urine collection container 14. In such examples, the pump 16 may be disposed between the fluid collection device 12 and the urine collection container 14.

The urine collection container 14 is sized and shaped to retain a fluid therein. The urine collection container 14 may include a bag (*e.g*., drainage bag), a bottle, a canister, or a cup (*e.g*., collection jar), or any other enclosed container for storing bodily fluid(s) such as urine. In some examples, the conduit 17 may extend from the fluid collection device 12 and attach to the urine collection container 14 at a first point therein. An additional conduit 17 may attach to the urine collection container 14 at a second point thereon and may extend and attach to the pump 16. Accordingly, a vacuum (*e.g*., suction) may be drawn through fluid collection device 12 via the urine collection container 14. Fluid, such as urine, may be drained from the fluid collection device 12 using the pump 16.

The pump 16 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 16 may provide a vacuum or suction to remove fluid from the fluid collection device 12. In some examples, the pump 16 may be powered by one or more of a power cord (*e.g.,* connected to a power socket), one or more batteries, or even manual power (*e.g.,* a hand operated vacuum pump). In some examples, the pump 16 may be sized and shaped to fit outside of, on, or within the fluid collection device 12. For example, the pump 16 may include one or more miniaturized pumps or one or more micro pumps. The vacuum sources disclosed herein may include one or more of a switch, a button, a plug, a remote, or any other device suitable to activate the pump 16.

Fluid collection devices described herein include a fluid impermeable barrier and a body that is at least partially fluid permeable. The fluid impermeable barrier includes a distal end region and a proximal end region defining an aperture sized and dimensioned to have urine drawn therethrough by a vacuum source. The elongated body has a portion of the body that is not covered by the fluid impermeable barrier between the distal end region and the proximal end region (*e.g.,* the fluid impermeable barrier is absent between at least a portion of the distal end region and the proximal end region of the elongated body, or at least a portion of the elongated body between the distal end region and the proximal end region of the elongated body is free from covering by the fluid impermeable barrier) and positioned on the fluid collection device to receive urine discharged from a urethra at least proximate to the portion of the body. The fluid collection devices may include at least one of the fluid impermeable barrier being an injection molded fluid impermeable barrier. The body includes an extruded support.

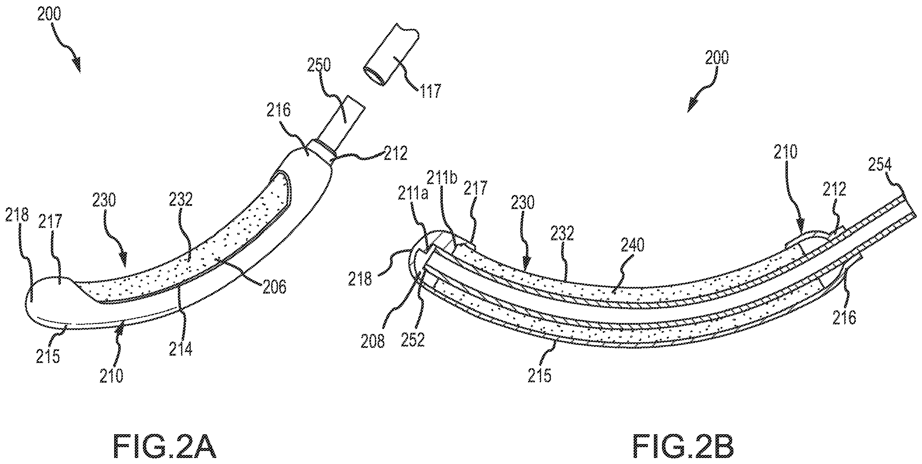

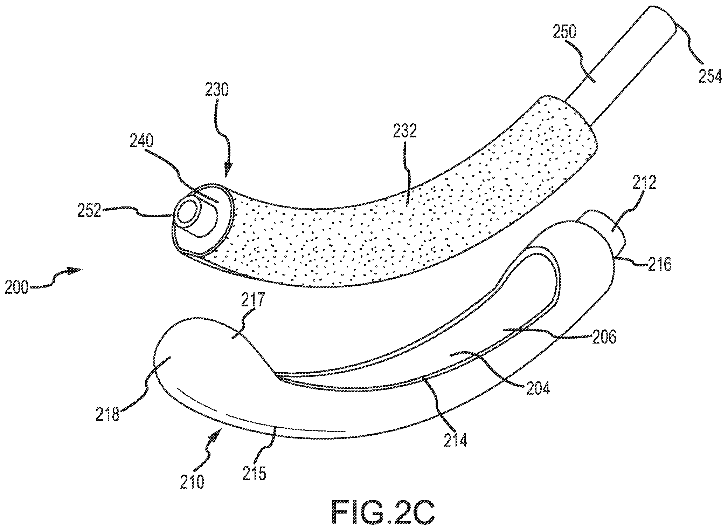

Turning now to **FIGS. 2A-2C****,** which do not show the slot and side slot structure of the presently claimed invention, a fluid collection device may include a fluid collection device may include a fluid impermeable barrier 210 and a fluid permeable body 230. The fluid impermeable barrier 210 may be elongated and include a distal region 218, a proximal end region 216, an aperture 212 or port positioned at the proximal end region 216 and sized and dimensioned to urine drawn therethrough by a vacuum source. The fluid impermeable barrier 210 may include a back side 215 and a front side 217. The front side 217 is positioned to be oriented towards the user when the fluid collection device 200 is in use. The fluid impermeable barrier 210 may at least partially define a chamber 204 and an opening 206 on the front side 217 in fluid communication with the chamber 204 and positioned on the fluid collection device 200 to receive urine discharged form a urethra that is at least proximate (*e.g*., adjacent) to the opening 206. An inward border or edge 214 of the fluid impermeable barrier 210 defines the opening 206. The edge 214 may include two or more opposing arced portions, the arced portions following the outer circumference or periphery of the fluid impermeable barrier 210.

The fluid impermeable barrier 210 also may at least partially define a reservoir 208 (*e.g.,* sump) in the chamber 204 at the distal end region 218 of the fluid impermeable barrier 210. The reservoir 208 may be positioned for fluid collection in the fluid collection device 200 before withdraw from the fluid collection device 200 through a conduit 250 having an inlet 252 positioned proximate to the reservoir 208. The reservoir 208 may be an at least partially (*e.g*., entirely) unoccupied portion of the chamber 204 and is at least partially (*e.g*., entirely) void of other material. The reservoir 208 may be substantially filled with one or more fluid permeable materials described herein. The reservoir 208 is defined at least partially by a terminating end the fluid permeable body 230 (shown in **FIG. 2B****)** and the distal end region 218 of the fluid impermeable barrier 210.

The fluid impermeable barrier 210 may include one or more protrusions 211a positioned within the chamber 204 at the distal end region 218 to space at least a portion of the inlet 252 from the distal end region 218 of the fluid impermeable barrier 210 effective to allow fluid communication between the chamber and the inlet 252. For example, the fluid impermeable barrier 210 may include a protrusion 211a at the distal end region 218 protruding into the reservoir 208. The protrusion 211a may be sized smaller than the inlet 252 such that if the conduit 250 is pushed too far into the chamber 204, the protrusion 211a may contact only a portion (*e.g.,* a front portion) of the inlet 252 of the conduit 250, but also space a portion of the inlet 252 from the distal end region 218 of the fluid impermeable barrier 210. This space between the inlet 252 and the distal end region 218 of the fluid impermeable barrier 210 allows fluid to still be pulled into the conduit 250 even when the protrusion 211a is contacting a portion of the inlet 252. The protrusion 211a then, results in the technical effect of preventing the conduit 250 from being blocked or plugged if the conduit 250 is pushed too far into the chamber 204.

The fluid impermeable barrier 210 may include one or more protrusions 211b positioned within the chamber 204 at the distal end region 218 to space at least a portion of the fluid permeable body 230 from the distal end region 218 of the fluid impermeable barrier 210. For example, the fluid impermeable barrier 210 may include a protrusion 211b at the distal end region 218 protruding into the reservoir 208. The protrusion 211b may be sized smaller than the distal end of the fluid permeable body 230 such that if the fluid permeable body 230 is pushed too far into the chamber 204, the protrusion 211b may contact only a portion (*e.g.,* a front portion) of the fluid permeable body 230, but also space a portion of fluid permeable body 230 from the distal end region 218 of the fluid impermeable barrier 210. This space between the fluid permeable body 230 and the distal end region 218 of the fluid impermeable barrier 210 allows the reservoir 208 to remain void or open for urine collection.

The fluid impermeable barrier 210 may be substantially cylindrical. The fluid impermeable barrier 210 may include other geometrical shapes and configurations. The fluid impermeable barrier 202 may be formed of any suitable fluid impermeable materials, such as a fluid impermeable polymer (*e.g*., silicone, polypropylene, polyethylene, polyethylene terephthalate, a polycarbonate, etc.), polyurethane films, thermoplastic elastomer (TPE), rubber, thermoplastic polyurethane (TPU), another suitable material, or combinations thereof. The fluid impermeable barrier 210 is flexible, thereby enabling the fluid collection device 200 to bend or curve when positioned against the body of a wearer. Example fluid impermeable barriers may include, but are not limited to, a fluid impermeable barrier including at least one of Versaflex CL 2000X TPE, Dynaflex G6713 TPE, or Silpuran 6000/05 A/B silicone. The fluid impermeable barrier 210 may be air permeable. The fluid impermeable barrier 210 may be formed of a hydrophobic material that defines a plurality of pores. One or more portions of at least the outer surface of the fluid impermeable barrier 210 may be formed from a soft and/or smooth material, thereby reducing chaffing.

The fluid impermeable barrier 210 may be molded, such as injection molded. For example, the fluid impermeable barrier 210 may be injection molded from one or more of silicone or a polymer such as TPE or TPU. The fluid impermeable barrier 210 may be injection molded as one component. The fluid impermeable barrier 210 may be injection molded to be curved or arced longitudinally. Injection molding the fluid impermeable barrier 210 may reduce the cost of manufacturing the fluid impermeable barrier 210 and the fluid collection device 200. For example, injection molding the fluid impermeable barrier 210 results in the technical effect of reducing the material cost and provide a faster cycle time for manufacturing the fluid impermeable barrier 210. Injection molding the fluid impermeable barrier 210 results in the technical advantage of repeatability and consistency. Injection molding the fluid impermeable barrier 210 may result in the technical advantage of incorporating features described herein into a single body.

The fluid collection device 200 also includes the fluid permeable body 230 positioned within the chamber 204 and extending at least partially across the opening 206 and at least partially between the distal end region 217 and the proximal end region 216. The fluid permeable body 230 may be shaped generally complementary to the shape of the chamber 204 of the fluid impermeable barrier 210. The fluid permeable body 230 fills substantially all of the chamber 204, except for the reservoir 208.

The fluid permeable body 230 can be configured to wick and/or allow transport of fluid away from the opening 206 towards the reservoir 208 and/or the inlet 252. More specifically, the fluid permeable body 230 can be configured to wick and/or allow transport of fluid away from the opening 206 toward the reservoir 208 and the inlet 252. The fluid permeable body 230 can include any material that can wick and/or allow transport of the fluid. The permeable properties referred to herein can be wicking, capillary action, diffusion, or other similar properties or processes, and are referred to herein as "permeable" and/or "wicking." Such "wicking" or other physical properties may exclude absorption into the fluid permeable body 230, such as not include adsorption of the bodily fluids into the fluid permeable body 230. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the fluid permeable body 230 (*e.g.,* absorbency), such as less than about 30 wt% of the dry weight of the fluid permeable body 230, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the fluid permeable body 230. In an embodiment, the fluid permeable body 230 may include at least one absorbent or adsorbent material.

The fluid permeable body 230 can include a one-way fluid movement fabric. As such, the fluid permeable body 230 can remove fluid from the area around the urethra, wound, and/or skin, thereby leaving the area dry. The fluid permeable body 230 can enable the fluid to flow generally towards the reservoir 208 and the inlet 252. The fluid permeable body 230 can include a porous or fibrous material, such as hydrophilic polyolefin. In some embodiments, the fluid permeable body 230 consists of or consists essentially of a porous or fibrous material, such as hydrophilic polyolefin. Examples of polyolefin that can be used in the fluid permeable body 230 include, but are not limited to, polyethylene, polypropylene, polyisobutylene, ethylene propylene rubber, ethylene propylene diene monomer, or combinations thereof. Moreover, the fluid permeable body 230 can be manufactured according to various manufacturing methods, such as molding, extrusion, or sintering. For example, the fluid permeable support 240 of the fluid permeable body 230 may be formed through extrusion. The fluid permeable support 240 may include open or closed cell foam extrusion. The fluid permeable body 230 can include varying densities or dimensions.

The fluid permeable body 230 can include two or more layers of fluid permeable materials. For example, the fluid permeable body 230 can include a fluid permeable membrane 232 covering or wrapped around a fluid permeable support 240, with both the fluid permeable membrane and the fluid permeable support being disposed at least partially in the chamber 204. The fluid permeable membrane 232 can cover or extend across at least a portion (*e.g*., all) of at least the side of the fluid permeable support 240 facing the urethra of the user. The fluid permeable membrane 232 and the fluid permeable support 240 can be configured to wick any fluid away from the opening 206, thereby preventing the fluid from escaping the chamber 204 through the opening 206 and promoting removal of the fluid from the chamber 204 through the conduit 250. The permeable properties referred to herein can be wicking, capillary action, diffusion, or other similar properties or processes, and are referred to herein as "permeable" and/or "wicking."

The fluid permeable membrane 232 and the fluid permeable support 240 also can wick and/or allow transport of the fluid generally towards the reservoir 208. The fluid permeable membrane 232 can include any material that can wick the fluid. For example, the fluid permeable membrane can include fabric, such as a gauze (*e.g.,* a silk, linen, polymer based materials such as polyester, or cotton gauze), nylon (such as a spun nylon fibers), another soft fabric (*e.g*., jersey knit fabric or the like), or another smooth fabric (*e.g.,* rayon, satin, or the like). Forming the fluid permeable membrane from gauze, soft fabric, and/or smooth fabric can reduce chaffing caused by the fluid collection device 200. Other embodiments of fluid permeable membranes and fluid permeable supports are disclosed in U.S. Patent Application No. 15/612,325 filed on June 2, 2017; U.S. Patent Application No. 15/260,103 filed on September 8, 2016; U.S. Patent Application No. 15/611,587 filed on June 1, 2017; PCT Patent Application No. PCT/US19/29608, filed on April 29, 2019. The fluid permeable body 230 may include a fluid permeable support 240 including a porous spun nylon fiber structure and a fluid permeable wicking membrane 232 including gauze at least partially enclosing the spun nylon fiber structure. For example, the fluid permeable body 230 may include a gauze or other wicking fabric positioned to contact the skin of the user through the opening 206. The gauze or other wicking fabric may be wrapped around a body of spun nylon fibers material and/or covering both sides of a substantially planar spun nylon fibers material. The gauze or other wicking fabric covers the side of substantially planar spun nylon fibers material that is oriented towards the skin of the user.

The fluid collection device 200 also includes the conduit 250 that is at least partially disposed in the chamber 204. The conduit 250 *(e.g.,* a tube) may provide fluid communication between an interior region of the chamber 204 (*e.g.,* the reservoir 208) and a fluid storage container (not shown) or a vacuum source (not shown). For example, the conduit 250 may directly or indirectly fluidly couple the interior region of the chamber 204 with the fluid storage container or the portable vacuum source. The conduit 250 includes the inlet 252 positioned within the chamber 204 proximate to the reservoir 208 and/or the distal end region 218 of the fluid impermeable barrier 210. The inlet 252 may be substantially flush with the distal end of the fluid permeable body 230, extending at least partially from the fluid permeable body 230, or recessed from the fluid permeable body 230. The conduit 250 extends through the aperture 212 and includes an outlet 254 positioned outside the chamber 204. The outlet 254 may be configured to connect with an additional conduit 117 in fluid communication with a vacuum source effective to provide fluid communication between the conduit 250 and the additional conduit 117.

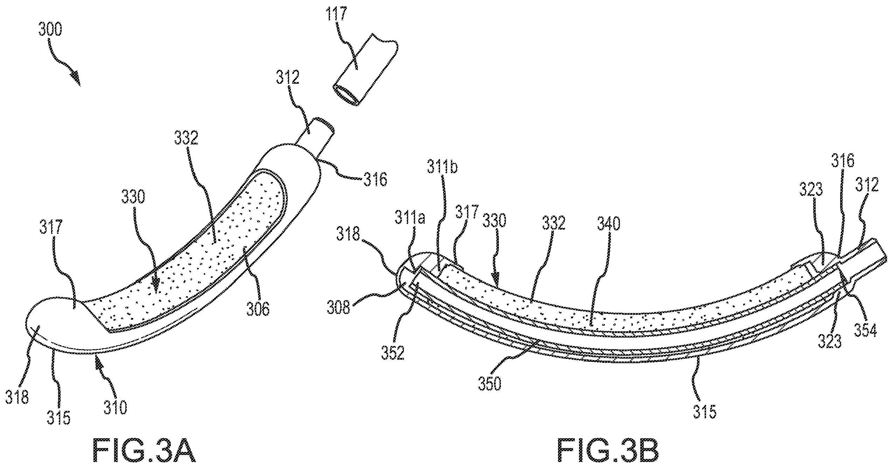

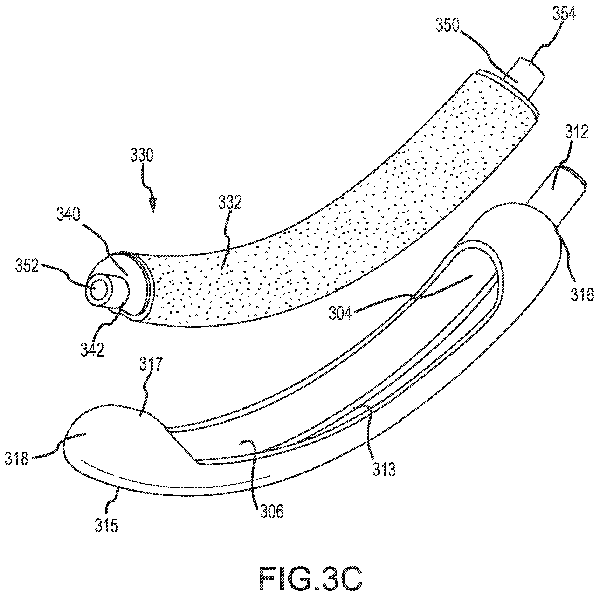

Turning to **FIGS. 3A-3C****,** which do not show the slot and side slot structure of the presently claimed invention, a fluid collection device may include a fluid collection device 300 may include a fluid impermeable barrier 310 and a fluid permeable body 330. Unless otherwise noted, the fluid collection device 300 may include any aspect of the fluid collection device 200. For example, the fluid collection device 300 may include the fluid impermeable barrier 310 having a proximal end region 316 and a distal end region 318 and defining a chamber 304 and an opening 306 on a front side 317 of the fluid impermeable barrier 310. The fluid collection device 300 may include one or more protrusions 311a, 311b and a reservoir 308 of void space at the distal end region 318 of the fluid impermeable barrier 310. The fluid collection device 300 also may include the fluid permeable body 330 including a fluid permeable membrane 332 and a fluid permeable support 340.

The fluid impermeable barrier 310 may include an aperture 312 defined by an external port at the proximal end region 316 of the fluid impermeable barrier 310. The external port is sized and dimensioned to detachably secure to an additional conduit 317 effective to provide fluid communication between the additional conduit 117 and the chamber 304. The fluid impermeable barrier 310 may define an internal port 323 positioned within the fluid collection device 300 at the proximal end region 316 of the fluid impermeable barrier 310 and in fluid communication with the aperture 312. The fluid collection device 300 also may include a conduit 350 partially positioned or positionable in the internal port 323 of the fluid impermeable barrier 310. For example, the conduit 350 may include an outlet 354 positioned or positionable in the internal port 323. The conduit 350 also may include an inlet 352 positioned in the chamber 304 proximate to the reservoir 308 in distal end region 318 of the fluid impermeable barrier 310.

The fluid impermeable barrier 310 may include a rear side 315 and a front side 317. The rear side 315 of the fluid impermeable barrier 310 may include an elongated planar portion extending at least partially between the proximal end region 316 and the distal end region 318. The front side 317 of the fluid impermeable barrier 310 may include an arced region at each of the distal end region 318 and the proximal end region 316. This configuration of the fluid impermeable barrier 310 results in the technical effect of using less material, thereby reducing costs of manufacturing the fluid collection device 300 while still providing the same effective fluid collection diameter. The fluid impermeable barrier 310 may include one or more internal longitudinal ribs 313 protruding into the chamber 304 and helping maintain shape of the fluid impermeable barrier 310. The one or more ribs 313 may include a material that is stiffer than the remainder of the fluid impermeable barrier 310.

The fluid permeable body 330 may include at least an elongated fluid permeable support 340 having an arced profile defining a channel 342 and complementary to the arced region at each of the distal end region 318 and the proximal end region 316. For example, the fluid permeable support 340 may include a cross-sectional profile that is semi- or half-circle shaped. The conduit 350 may be positioned at least partially (*e.g*., entirely) within the channel 342. The fluid permeable body 330 also includes a fluid permeable membrane 332 positioned around the fluid permeable support 340. The fluid permeable membrane 332 may be wrapped or positioned around the fluid permeable support 340 with the conduit 350 positioned within the channel 342 such that the conduit 350 is positioned between some of the fluid permeable support 340 and some of the fluid permeable membrane 332. The fluid permeable membrane 332 may help secure the conduit 350 in the channel 342. This configuration of the fluid permeable body 330 results in the technical effect of using less material, thereby reducing costs of manufacturing the fluid collection device 300.

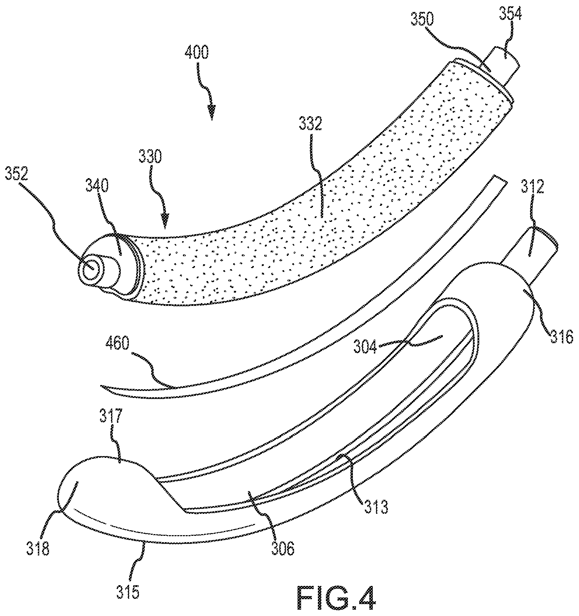

Turning to **FIG. 4****,** which does not show the slot and side slot structure of the presently claimed invention, a fluid collection device may include a fluid collection device 400 may include a support member 460 secured to the rear side of the fluid impermeable barrier 410. Unless otherwise noted, the fluid collection device 400 may include any aspect of the fluid collection devices 200, 300. Moreover, the support member 460 may be include any of fluid collection device 200, 300. The support member 460 may include a shape memory polymer or a metal. For example, the support member 460 may include a malleable metal strip or wire trapped between the fluid permeable body 330 and the fluid impermeable barrier 310.

At least the support member 460 may include a shape memory material such as a shape memory polymer or a metal (*e.g*., shape memory metal). The shape memory material may be secured to or embedded into at least a portion of the fluid impermeable barrier 310. Suitable shape memory materials are composed to adopt an intermediate or permanent shape in response to a stimuli. The stimuli may include an external physical force (*e.g*., bending force), heat, electrical bias, or a magnetic field. While the term "shape memory" is used to describe some of the "shape memory materials" herein, it should be understood that, in some examples, the material modified by the term "shape memory" may not necessarily need to return to a preselected shape upon application of a stimuli, as understood as the classical definition of the "shape memory material." Rather, at least some of the shape memory materials herein may simply hold a selected shape when bent, set, or cured into a specific shape and/or when cooled in a specific shape, regardless of the stimuli applied thereto after. The shape memory materials may be returned to the original shape or changed to a new shape by application of stimuli. For example, a metal wire bent to a first shape may be utilized as the shape memory material, whereinafter the metal wire may be modified to a second shape via physical force applied thereto or via heating.

The shape memory material may include metal, such as an elemental metal, an alloy, or shape memory alloy. Suitable shape memory metals may include standard steels, stainless steel, carbon alloy steel, head treated steel, aluminum, silver, copper, iron, nickel, zinc, tin, beryllium, or the like. Suitable shape memory alloys may include stainless steel; galvanized steel; aluminum alloys; nickel-titanium alloys, such as Nitinol, Ni-Ti-Cu, Ni-Ti, Co, or the like; copper-based alloys such as Cu-Zn-Al, Cu-Al-Ni, Cu-Al-Sn, or the like; Co-Cr-Ni-Mo alloys (*e.g.,* Elgiloy^{®}) or the like; or any other alloy having shape memory characteristics. As explained above, the shape memory metals or alloys may merely be metals or alloys that may be shaped to a selected configuration. In some materials, the shape memory metals or alloys may return to a primary shape when an external stimuli is applied thereto. In some materials, the outer surface of the shape memory metal may be coated with a polymer, anodized, passivated, or otherwise treated to prevent corrosion.

Shape memory polymers ("SMPs") may include polyurethane-based SMPs such as a copolymer (*e.g*., copolyester, polyurethane, polyetherester, etc.) including blocks of one or more of poly(ε-caprolactone), polyethyleneterephthalate (PET), polyethyleneoxide (PEO), polyethylene glycol (PEG), polystyrene, polymethylmethacrylate (PMMA), Polybutylmethacrylate (PBMA), poly(N,N-butadiene), poly(N-methyl-N-oxazoline), polytetrahydrofuran, or poly(butylene terephthalate); thermoplastic polymers such as polyether ether ketone (PEEK), nylon, acetal, polytetrafluoroethylene (PTFE), polysulphone, or the like; Polynorbonene; other deformable polymers; or any other shape memory polymer.

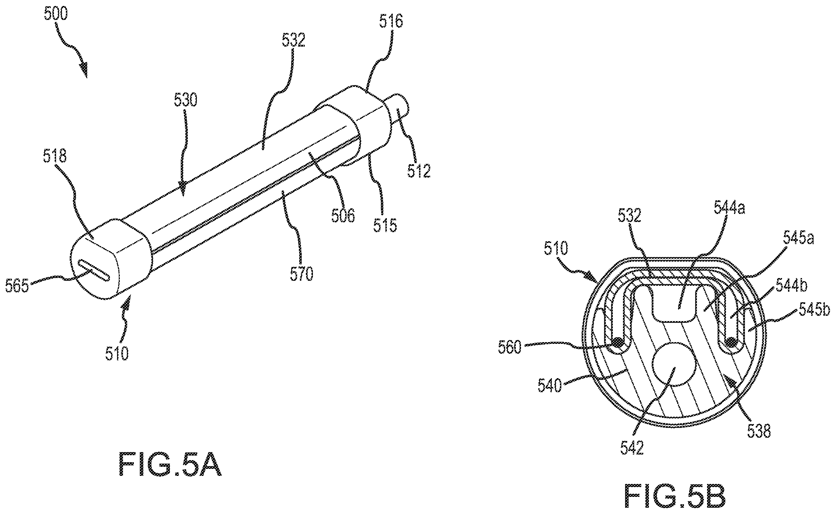

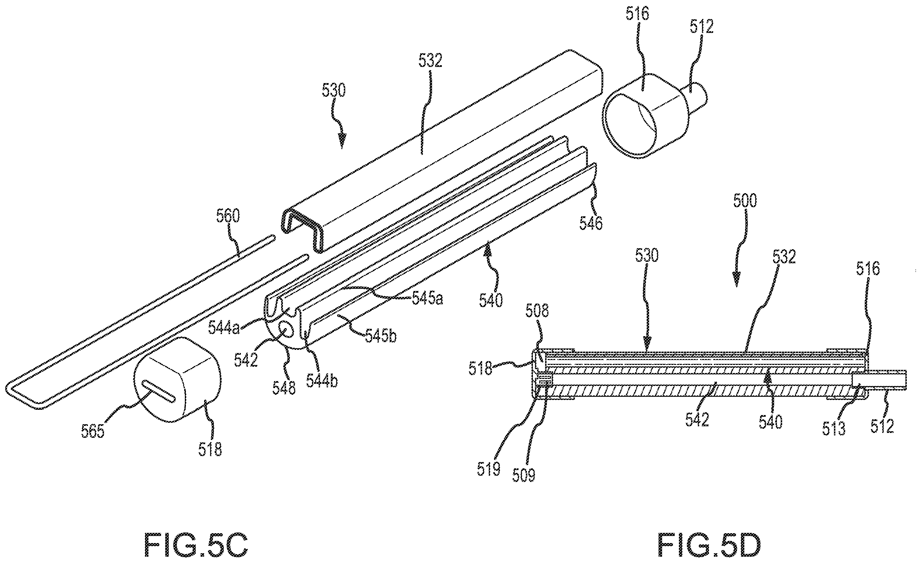

Turning now to **FIGS. 5A-5D****,** a fluid collection device 500 in accordance with the present invention includes a fluid impermeable barrier having a distal end region 518 and a proximal end region 516 defining an aperture 512 sized and dimensioned to have urine drawn therethrough by a vacuum source. The fluid collection device 500 includes an elongated body 530 that is at least partially fluid permeable and has a portion of the body 530 that is not covered by the fluid impermeable barrier between the distal end region 518 and the proximal end region 516 and that is positioned on the fluid collection device 500 to receive urine discharged from a urethra at least proximate to the portion of the body 530. The fluid impermeable barrier may cover only a portion of the elongated body 530 such that the fluid impermeable barrier is absent from covering the portion of the elongated body 530 between the distal end region 518 and the proximal end region 516. The portion of the elongated body 530 between the distal end region 518 and the proximal end region 516, then, may be free from covering by the fluid impermeable barrier.

The body 530 includes a fluid permeable membrane 532 and an extruded support 540. In some embodiments, the extruded support 540 may include a foamed or soft material extrusion, such as a closed cell foam extrusion. The extruded support 540 may be generally fluid impermeable and support the fluid permeable membrane 532 of the body 530. The fluid permeable membrane 532 may include any aspect of the fluid permeable body 230 described above, such as a spun nylon fibers fluid permeable membrane. The fluid permeable membrane 532 may cover at least a portion of the extruded support 540, as described in greater detail below.

The extruded support 540 includes a distal end region 548, a proximal end region 546, and at least one slot 544a extending longitudinally on the extruded support 540. More particularly, the extruded support 540 may include a rear side and a front side including two side slots 544b extending longitudinally on the front side of the extruded support with the slot 544a being positioned between the two side slots 544b. The extruded support 540 may include a plurality of longitudinal ridges at least partially defining the slots 544a, 544b. For example, the slot 544a may be defined at least partially by the longitudinal ridges 545a. The side slots 544b may be defined at least partially by one of the longitudinal ridges 545a and a side longitudinal ridge 545b. The extruded support 540 includes a channel 542 extending longitudinally therethrough. The channel 542 may be completely enclosed within the extruded support 540 such that the extruded support 540 surrounds and/or defines the channel 542. For example, the channel 542 may include an O- or D-shaped channel extending longitudinally through the extruded support 540 from the distal end region 548 to the proximal end region 546.

In some embodiments, the fluid permeable membrane 532 is secured or securable to the extruded support 540 such that a portion of the fluid permeable membrane 532 extends across the at least one slot 544a on the front side of the extruded support 540. The portion of the fluid membrane 532 that extends across the at least one slot 544a may include the portion of the body 530 that is not covered by the fluid impermeable barrier (*e.g.,* the fluid impermeable barrier is absent) between the distal end region 518 and the proximal end region 516 and is positioned on the fluid collection device 500 to receive urine discharged from a urethra. A portion of the fluid permeable membrane 532 may be positioned within the two side slots 544b. For example, the fluid collection device 500 may include a shape memory polymer or metal 560 positioned within each of the two side slots 544b and securing the portion of the fluid permeable membrane 532 positioned within the two side slots 544b. In some embodiments, the fluid permeable membrane 532 may include at least a partial loop within each of the two side slots 544b and the shape memory polymer or metal 560 includes an elongated U-shaped shape memory polymer or metal extending through the distal end region 518 and into the at least partial loop in each of the two side slots 544b.

The fluid impermeable barrier of the fluid collection device 500 includes two caps. The distal end region 518 of the fluid impermeable barrier includes a distal end cap 518 and the proximal end region 516 of the fluid impermeable barrier includes a proximal end cap 516. At least of portion of the distal end cap 518 and the proximal end cap 516 may be shaped complementary to the profile of the body 530. At least a portion of the distal end region 548 of the extruded support 540 may be positioned in the distal end cap 518 and at least a portion of the proximal end region 546 of the extruded support 540 may be positioned in the proximal end cap 516. A reservoir 508 of void space may be positioned between the distal end region 548 of the extruded support 540 and at least a portion of the distal end cap 518 effective to provide fluid communication between the at least one slot 544a and the channel 542 of the extruded support 540. The reservoir 508 may be at least partially defined by portions of each of the distal end region 548 of the extruded support 540 and the distal end cap 518.

In some embodiments, the distal end cap 518 has one or more tabs on the inner surface positioned to prevent or inhibit the extruded support 540 from being pushed too far into the distal end cap 518 that would prevent fluid communication between the at least one slot 544a and the channel 542. Instead, the one or more tabs ensure a reservoir 508 is formed in the distal end cap 518 and fluid communication is maintained between the at least one slot 544a and the channel 542. In some embodiments, the distal end cap 518 includes an alignment feature 519 (*e.g.,* boss) extending partially into the channel 542 at the distal end region 548 of extruded support 540. The alignment feature 519 may be shaped complementary to at least a portion of the channel 542 and may include one or more slits 509, openings, apertures, or perforations configured to provide fluid communication between the channel 542 and the reservoir 508. In some embodiments, at least one of the alignment feature 519 or the channel 542 includes one or more tabs or stops positioned on the alignment feature 519 and/or the channel 542 to prevent the alignment feature 519 from being inserted too far into the channel 542. The one or more tabs or stops in at least one, some, or all embodiments, then, result in the technical effect of insuring fluid communication between the channel 542 and the reservoir is maintained. In some embodiments, the distal cap 518 includes wire sockets for a U-shaped wire shape memory polymer or metal 560 or a socket for a malleable strip. For example, the U-shaped wire may include an elongated staple that extends through at least the distal end cap 518. In some embodiments, the elongated staple may extend through the distal end cap 518 and the proximal end cap 516, and may be crimped into the proximal end cap 516. In some embodiments, the distal end cap 518 and the proximal end cap 516 may be bonded to the extruded support 540 or secured to the body 530 with an adhesive.

In some embodiments, the proximal end cap 516 at least partially defines the aperture 512. The aperture 512 may be defined by a portion of the proximal end cap 516 extending outward from the proximal end cap 516 and configured to detachably secure to a conduit (*e.g*., tube). The proximal end cap 516 also may include an internal port 513 extending partially into the channel 542 in the proximal end region 546 of the extruded support 540. The end caps 516, 518 may include any fluid impermeable material described above in relation to the fluid impermeable barrier 210. In some embodiments, the end caps 516, 518 include a TPE material.

In use, the fluid collection device 500 may be positioned with the fluid permeable membrane 532 covering the at least one slot 544a at least proximate (*e.g*., adjacent) to the urethra of the user. The ridges 545a on the extruded support 540 may support the fluid permeable membrane 532 such that the at least one slot 544a remains open and void of material (other than urine). With a vacuum creating a negative pressure on the fluid collection device 500, urine discharged on the fluid permeable membrane may be drawn or otherwise enter the at least one slot 544a and flow to the reservoir 508. The vacuum may draw the urine in the reservoir 508 through the alignment feature 519, into the channel 542, through the aperture 512, and out of the fluid collection device 500.

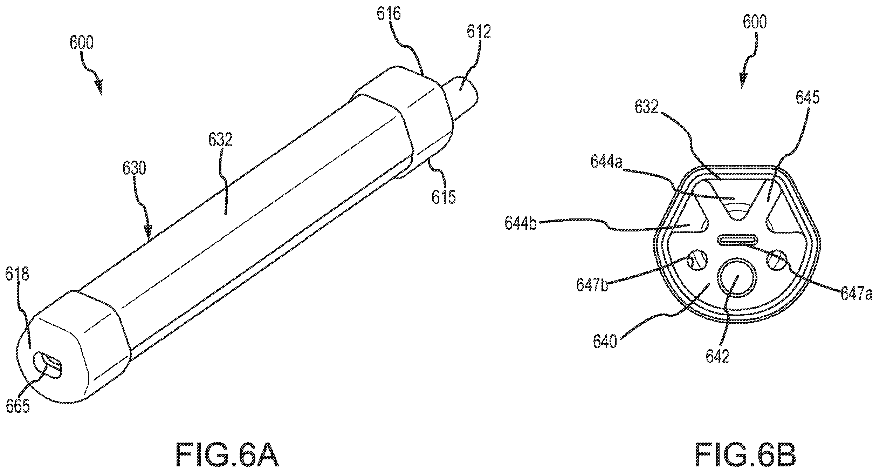

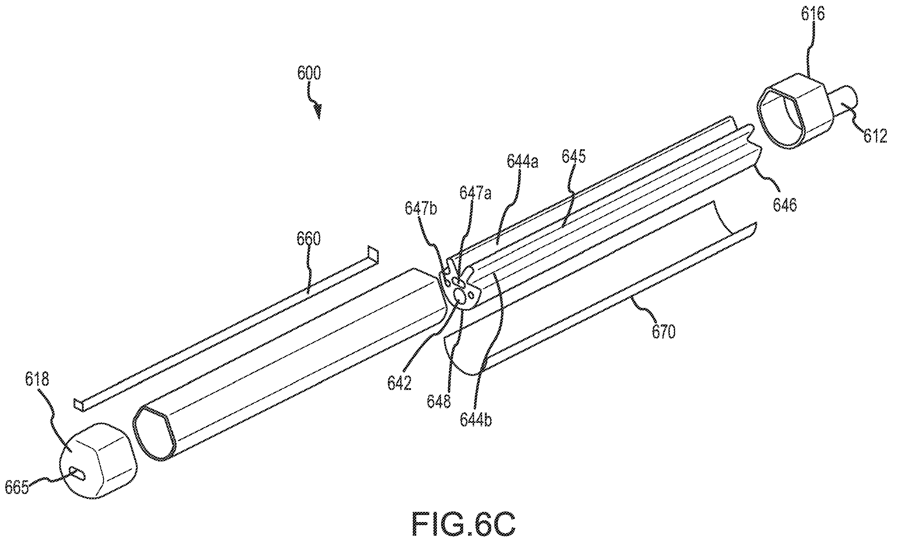

In some embodiments, a fluid collection device may include a label on the fluid collection device that includes instructions, product identification, and/or user-notated usage data. Turning to **FIGS. 6A-6C****,** a fluid collection device 600 in accordance with the invention includes a fluid impermeable barrier having a distal end region 618 and a proximal end region 616 defining an aperture 612 sized and dimensioned to have urine drawn therethrough by a vacuum source. The fluid collection device 600 includes an elongated body 630 that is at least partially fluid permeable and has a portion of the body 630 that is not covered by the fluid impermeable barrier (*e.g*., the fluid impermeable barrier is absent) between the distal end region 618 and the proximal end region 616 and is positioned on the fluid collection device 600 to receive urine discharged from a urethra at least proximate to the portion of the body 230. A label 670 may be secured to the body 630 distal to the portion of the body 630 that is not covered by the fluid impermeable barrier between the distal end region 618 and the proximal end region 616. Unless otherwise noted, the fluid collection device 600 may include any aspect of the fluid collection device 500.

The body 630 includes a fluid permeable membrane 632 and an extruded support 640. In some embodiments, the extruded support 640 may include a foamed or soft material extrusion, such as a closed cell foam extrusion. The extruded support 640 may be generally fluid impermeable and support the fluid permeable membrane 632 of the body 630. The fluid permeable membrane 632 may include any aspect of the fluid permeable body 230 described above, such as a spun nylon fibers fluid permeable membrane. The fluid permeable membrane 632 may cover at least a portion of the extruded support 640, as described in greater detail below.

The extruded support 640 includes a distal end region 648, a proximal end region 646, and at least one slot 644a extending longitudinally on the extruded support 640. More particularly, the extruded support 640 includes a rear side and a front side including multiple slots 644a, 644b. The multiple slots include two side slots 644b and a slot 644a positioned between the two side slots 644b. Each of the multiple slots 644a, 644b may have a center angled relative to the center of other slots of the multiple slots 644a, 644b. The extruded support 640 may include a plurality of longitudinal ridges 645 at least partially defining the multiple slots 644a, 644b.

The extruded support 640 includes a channel 642 extending longitudinally therethrough. The channel 642 may be completely enclosed within the extruded support 640 such that the extruded support 640 surrounds and/or defines the channel 642. For example, the channel 642 may include an O- or D-shaped channel extending longitudinally through the extruded support 640 from the distal end region 648 to the proximal end region 646. The extruded support 640 also may include one or more lumens 647a, 647b extending longitudinally at partially through the extruded support 640 and positioned to receive a shape memory polymer or metal 660. For example, the extruded support 640 may include a lumen 647a sized and dimensioned to receive a malleable polymer or metal and/or two lumens 647b sized and dimensioned to receive arms or prongs of a U-shaped malleable polymer or metal wire. In some embodiments, the distal end cap 618 and the proximal end cap 616 may be bonded to the extruded support 640 or secured to the body 630 with an adhesive.

In some embodiments, the fluid permeable membrane 632 is secured or securable to the extruded support 640 such that a portion of the fluid permeable membrane 632 extends across the multiple slots 644a, 644b on the front side of the extruded support 640. The portion of the fluid membrane 632 that extends across the multiple slots 644a, 644b may include the portion of the body 630 that is not covered by the fluid impermeable barrier between the distal end region 618 and the proximal end region 616 and is positioned on the fluid collection device 600 to receive urine discharged from a urethra. In some embodiments, the fluid permeable membrane 632 may wrap around the periphery of the extruded support 640. For example, the fluid permeable membrane 632 may be generally tubular when wrapped around the periphery of the extruded support 640. The ridges 645 on the extruded support 640 may support the fluid permeable membrane 632 such that the multiple slots 644a, 644b remain open and void of material (other than urine). When wrapped around the extruded support 640, the portion of the fluid permeable membrane 632 not covered by the fluid impermeable barrier may include a substantially planar center region covering the slot 644a and two substantially planar side regions covering the slots 644b and angling towards the substantially planar center region.

The fluid impermeable barrier of the fluid collection device 600 includes two caps. The distal end region 618 of the fluid impermeable barrier includes a distal end cap 618 and the proximal end region 616 of the fluid impermeable barrier includes a proximal end cap 616. At least of portion of the distal end cap 618 and the proximal end cap 616 may be shaped complementary to the profile of the body 630. At least a portion of the distal end region 648 of the extruded support 640 may be positioned in the distal end cap 618 and at least a portion of the proximal end region 646 of the extruded support 640 may be positioned in the proximal end cap 616. A reservoir (not shown) of void space may be positioned between the distal end region 648 of the extruded support 640 and at least a portion of the distal end cap 618 effective to provide fluid communication between the multiple slots 644a, 644b and the channel 642 of the extruded support 640. The reservoir may be at least partially defined by portions of each of the distal end region 648 of the extruded support 640 and the distal end cap 618, similar to the reservoir 508 of the fluid collection device 500.

In some embodiments, the distal end cap 618 has one or more tabs on the inner surface positioned to prevent or inhibit the extruded support 640 from being pushed too far into the distal end cap 618 that would prevent fluid communication between the multiple slots 644a, 644b and the channel 642. Instead, the one or more tabs ensure the reservoir is formed in the distal end cap 618 and fluid communication is maintained between the multiple slots 644a, 644b and the channel 642. In some embodiments, the distal end cap 618 includes an alignment feature (not shown) similar or the same as the alignment feature on the distal end cap 518 of the fluid collection device 500. In some embodiments, at least one of the alignment feature or the channel 642 includes one or more tabs or stops positioned on the alignment feature and/or the channel 642 to prevent the alignment feature from being inserted too far into the channel 642. The one or more tabs or stops, then, may result in insuring fluid communication between the channel 642 and the reservoir is maintained. The distal end cap 618 may include a receiver for the shape memory polymer or metal 660. For example, the distal end cap 618 may include a socket 665 for a strip or U-shaped wire shape memory polymer or metal 660.

In some embodiments, the proximal end cap 616 at least partially defines the aperture 612. The aperture 612 may be defined by a portion of the proximal end cap 616 extending outward from the proximal end cap 516 and configured to detachable secure to a conduit (e.g., tube). The proximal end cap 616 also may include an internal port (not shown) extending partially into the channel 642 in the distal end region 646 of the extruded support 640. The end caps 616, 618 may include any fluid impermeable material described above in relation to the fluid impermeable barrier 210. In some embodiments, the end caps 616, 618 include a TPE material.

In some embodiments, the fluid impermeable barrier of the fluid collection device 600 includes a substantially fluid impermeable sheet 670 secured to a portion of the body 630 with an adhesive and extending at least partially between the proximal end cap 616 and the distal end cap 618. The fluid impermeable sheet 670 may include a waterproof label and/or adhesive configured to prevent fluid transfer around the periphery of the body 630. In some embodiments, the fluid impermeable sheet 670 may include any fluid impermeable material described above in relation to the fluid impermeable barrier 210, such as TPE. In some embodiments, the fluid impermeable sheet 670 may be sprayed coated on the fluid permeable membrane 632. The fluid impermeable sheet 670 also may include artwork, instructions, product identification, and/or user-notated usage data. The incorporation of labeling in at least one, some, or all embodiments results in the technical effect of reducing packaging required for the fluid collection device 600, providing information or instructions to the user in a place that is less likely to be discarded by the user, and/or providing a space to record relevant data pertaining to fluid collection of the user. The fluid permeable sheet 670 may be adhered to the fluid permeable membrane 632 before securing the body 630 to the proximal end cap 616 and/or the distal end cap 618.

In use, the fluid collection device 600 may be positioned with the fluid permeable membrane 632 that covers the multiple slots 644a, 644b at least proximate (e.g., adjacent) to the urethra of the user. The ridges 645 on the extruded support 640 may support the fluid permeable membrane 632 such that the multiple slots 644a, 644b remain open and void of material (other than urine). With a vacuum creating a negative pressure on the fluid collection device 600, urine discharged on the fluid permeable membrane 632 may be drawn or otherwise enter one or more of the slots 644a, 644b and flow to the reservoir. The vacuum may draw the urine in the reservoir through the alignment feature, into the channel 642, through the aperture 612, and out of the fluid collection device 600.

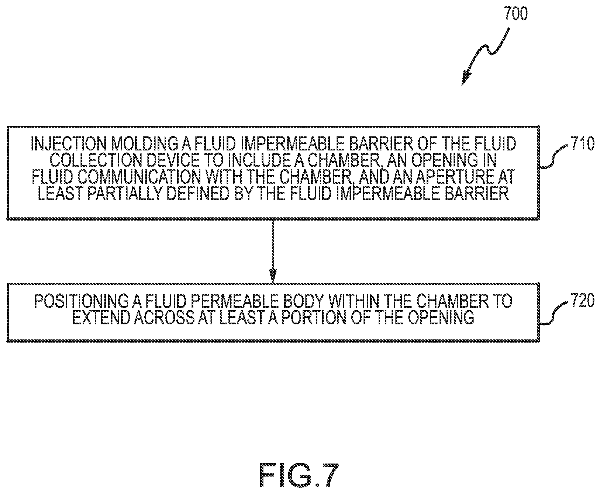

**FIG. 7** is a flow diagram of a non-claimed method 700 of manufacturing a fluid collection device. The method 700 includes an act 710 of injection molding a fluid impermeable barrier of the fluid collection device to include a chamber, an opening in fluid communication with the chamber, and an aperture at least partially defined by the fluid impermeable barrier. The opening may be positioned on the fluid collection device to receive urine discharged from a urethra at least proximate to the opening and the aperture may be sized and dimensioned to have urine drawn therethrough by a vacuum source. The act 710 of injection molding a fluid impermeable barrier of the fluid collection device may include injection molding the fluid impermeable barrier including at least one of thermoplastic polyurethane or thermoplastic elastomer. The method 700 also includes an act 720 of positioning a fluid permeable body within the chamber to extend across at least a portion of the opening.

The method 700 may further include positioning an inlet of a conduit at least partially within chamber. The act 710 of injection molding a fluid impermeable barrier of the fluid collection device may include injection molding the fluid impermeable barrier to include a top side defining the opening, a rear side opposite to the front end, a proximal end region defining the aperture, a distal end region opposite to the proximate end region, and one or more protrusions positioned within the chamber at the distal end region to space at least a portion of the inlet from the distal end region of the fluid impermeable barrier. Injection molding the fluid impermeable barrier may include injection molding the fluid impermeable barrier to be substantially cylindrical and positioning an inlet of a conduit at least partially within chamber may include inserting the conduit at least partially through the fluid permeable body and the aperture.

Injection molding the fluid impermeable barrier may include injection molding the fluid impermeable barrier to (1) define an internal port positioned within the fluid collection device and in fluid communication with the aperture and (2) include an external port at the proximal end region defining the aperture. The external port may be sized and dimensioned to detachably secure to an additional conduit effective provide fluid communication between the additional conduit and the conduit. The act of positioning an inlet of a conduit at least partially within chamber may include positioning the inlet of the conduit at least partially within the chamber and positioning an outlet of the conduit within the port.

The act 710 of injection molding the fluid impermeable barrier may include injection molding the fluid impermeable barrier to include an elongated planar portion on the rear side extending at least partially between a proximal end region and a distal end region and an arced region on the front side at each of the distal end region and the proximal end region. The method 700 may further comprise an act of positioning the conduit in a channel of an elongated fluid permeable support of the fluid permeable body.

The method 700 may further include positioning a fluid permeable membrane around the fluid permeable support and the conduit before positioning the fluid permeable body within the chamber to extend across at least the portion of the opening. The method 700 may further include securing a support member to the rear side of the fluid impermeable barrier, the support member including a shape memory polymer or a metal.

The acts of the method 700 described above are for illustrative purposes. For example, the acts of the method 700 can be performed in different orders, split into multiple acts, modified, supplemented, or combined. One or more of the acts of the method 700 can be omitted from the method 700. Any of the acts of the method 700 can include manufacturing of any of the fluid collection devices and systems disclosed herein.

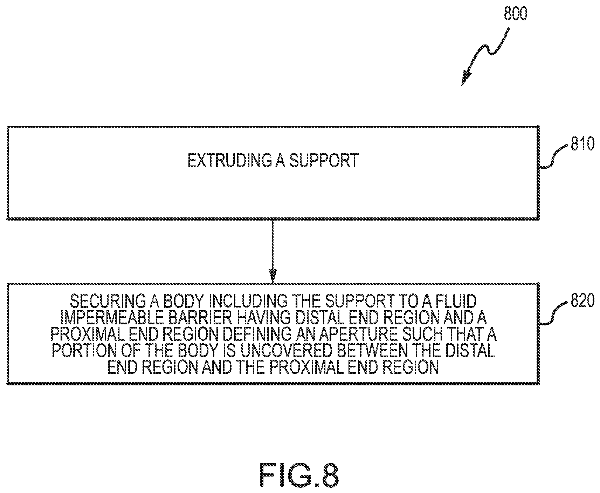

**FIG. 8** is a flow diagram of a non-claimed method 800 of manufacturing a fluid collection device. The method 800 includes an act 810 of extruding a support to include at least one slot extending longitudinally on the extruded support. Extruding a support includes extruding a closed cell foam extrusion forming the support. The method 800 also includes an act 820 of securing a body including the extruded support and that is at least partially fluid permeable to a fluid impermeable barrier having distal end region and a proximal end region defining an aperture such that a portion of the body is uncovered by the fluid impermeable barrier between the distal end region and the proximal end region and positioned on the fluid collection device to receive urine discharged from a urethra at least proximate to the portion of the body. The aperture is in fluid communication with the at least one slot and sized and dimensioned to have urine drawn therethrough by a vacuum source.

The method 800 includes an act of securing a fluid permeable membrane to the extruded support to form the body that is at least partially fluid permeable. The act 810 may include extruding the support to include a channel extending longitudinally therethrough. The method 800 may include an act of aligning the channel with the aperture of the fluid impermeable barrier before or when securing the body to a fluid impermeable barrier. The act 820 may include securing a proximal end cap to a proximal end region of the extruded support and securing a distal end cap to a distal end region of the extruded support to form a reservoir of void space between the distal end region of the extruded support and at least a portion of the distal end cap, the reservoir providing fluid communication between the at least one slot and the channel. The method 800 also may include securing an additional fluid impermeable barrier to a rear side of the extruded support with an adhesive before securing the distal end cap to the distal end region of the extruded support and securing the proximal end cap to the proximal end region of the extruded support. The additional fluid impermeable barrier may extend continuously between the distal end cap and proximal end cap when the body is secured to the fluid impermeable barrier.

The act 810 may include extruding the support to include a front side including two side slots extending longitudinally on the front side of the extruded support and the at least one slot extending longitudinally on the front side of the extruded support between the two side slots. The act 820 may include securing portions of the fluid permeable membrane in the two side slots with a shape memory polymer or metal extending through the two side slots, with a portion of the fluid permeable membrane extending at least partially across the front side of the extruded support between the two side slots to cover the one or more slots and include the portion of the body that is uncovered between the distal end region and the proximal end region of the fluid impermeable barrier. The act 820 also may include inserting an elongated U-shaped shape memory polymer or metal through the distal end cap and into the two side slots.

The act 810 includes extruding the support including a front side including three slots each having a center angled relative to the center of other slots of the three slots. The act 820 may include wrapping the fluid permeable membrane around the front side and the rear side of the extruded support such that a portion of the fluid permeable membrane covers the three slots to include the portion of the body that is uncovered between the distal end region and the proximal end region of the fluid impermeable barrier. The method 800 also may include inserting a shape memory polymer or metal to extend longitudinally through the distal end cap and at least a portion of the extruded support.

The method 800 also includes an act of inserting an alignment feature in the distal end cap into the channel at the distal end region of extruded support and inserting an internal port in the proximal end cap into the channel. The internal port may be in fluid communication with the aperture, and the alignment feature may include one or more slits providing fluid communication between the reservoir and the channel.

The acts of the method 800 described above are for illustrative purposes. For example, the acts of the method 800 can be performed in different orders, split into multiple acts, modified, supplemented, or combined. One or more of the acts of the method 800 can be omitted from the method 800. Any of the acts of the method 800 can include manufacturing of any of the fluid collection devices and systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

## Claims

1. A fluid collection device (500), comprising:
a fluid impermeable barrier having a distal end region (518) and a proximal end region (516) defining an aperture (512) sized and dimensioned to have urine drawn therethrough by a vacuum source; and
an elongated body (530) that is at least partially fluid permeable and has a portion of the elongated body between the distal end region and the proximal end region that is free from being covered by the fluid impermeable barrier and positioned to receive urine discharged from a urethra at least proximate to the portion of the elongated body, wherein the elongated body that is at least partially fluid permeable includes:
an extruded support (540) having a distal end region (548), a proximal end region (546), and at least one slot (544a) extending longitudinally on and in a longitudinal direction of the extruded support, and
a fluid permeable membrane (532) extending across and covering the at least one slot on the extruded support;
wherein the extruded support includes a channel (542) extending longitudinally therethrough and in fluid communication with the at least one slot and the aperture of the fluid impermeable barrier;
wherein the distal end region (518) of the fluid impermeable barrier includes a distal end cap (518) having at least a portion of the distal end region (548) of the extruded support (540) positioned therein, a reservoir (508) of void space being positioned between the distal end region of the extruded support and at least a portion of the distal end cap effective to provide fluid communication between the at least one slot and the channel;
the proximal end region of the fluid impermeable barrier includes a proximal end cap (516) having at least a portion of the proximal end region (546) of the extruded support (540) positioned therein and
wherein the extruded support (540) includes a rear side and a front side including two side slots (544b) extending longitudinally on and in a longitudinal direction of the front side of the extruded support, the at least one slot (544a) being positioned between the two side slots; and
respective portions of the fluid permeable membrane are positioned within each of the two side slots or extend across the two side slots on the front side of the extruded support.

2. The fluid collection device of claim 1, wherein:
the fluid impermeable barrier includes a substantially fluid impermeable sheet secured to a portion of the elongated body with an adhesive and extending between the proximal end cap and the distal end cap.

3. The fluid collection device of claim 1 or 2, wherein a portion of the fluid permeable membrane is positioned within each of the two side slots.

4. The fluid collection device of claim 3, further comprising a shape memory polymer or metal (560) positioned within each of the two side slots and securing the portion of the fluid permeable membrane positioned within the two side slots.

5. The fluid collection device of claim 4, wherein the shape memory polymer or metal includes an elongated U-shaped shape memory polymer or metal extending through the distal end cap and into the two side slots.

6. The fluid collection device of any one of the preceding claims, wherein the fluid permeable membrane is wrapped around the front side and the rear side of the extruded support such that a portion of the fluid permeable membrane covers the at least one slot and the two side slots and extends at least partially across the opening of the fluid impermeable barrier.

7. The fluid collection device of claim 6, further comprising a shape memory polymer or metal extending longitudinally through the distal end cap and at least a portion of the extruded support.

8. The fluid collection device of any of the preceding claims, wherein the distal end cap includes an alignment feature extending partially into the channel at the distal end region of extruded support and the proximal end cap includes an internal port extending partially into the channel, the alignment feature included one or more slits providing fluid communication between the reservoir and the channel.

9. The fluid collection device of any of the preceding claims, wherein the extruded support includes a closed cell foam extrusion.

10. The fluid collection device as claimed in claim 4 or any one of claims 5 to 9 as dependent upon claim 4, wherein the fluid permeable membrane (532) includes at least a partial loop within each of the two side slots (544b) and the shape memory polymer or metal (560) includes an elongated U-shaped shape memory polymer or metal extending through the distal end region (518) and into the at least partial loop in each of the side slots (544b).

11. The fluid collection device as claimed in any one of the preceding claims, wherein the distal end cap (518, 618) has one or more tabs on its inner surface positioned to prevent or inhibit the extruded support (540, 640) from being pushed too far into the distal end cap.

12. The fluid collection device as claimed in any one of the preceding claims, wherein the end cap (518, 618) includes an alignment feature (519) extending partially into the channel (542, 642) at the distal end region of the extruded support.

13. The fluid collection device as claimed in claim 12, wherein the alignment feature is configured to provide fluid communication between the channel and the reservoir.

14. The fluid collection device as claimed in claim 12 or 13, wherein the alignment feature includes one or more tabs or stops to prevent the alignment feature from being inserted too far into the channel (542, 642).

15. The fluid collection device as claimed in any one of the preceding claims, wherein the distal cap (518, 618) includes wire sockets for a U-shaped wire shape memory polymer or metal (560), or a socket (665) for a malleable strip (660).

## Patentansprüche

1. Flüssigkeitssammelvorrichtung (500), umfassend:
eine flüssigkeitsundurchlässige Barriere, die einen distalen Endbereich (518) und einen proximalen Endbereich (516) aufweist, die eine Öffnung (512) definieren, die so bemessen und dimensioniert ist, dass Urin von einer Vakuumquelle dort hindurch gesaugt werden kann; und
einen länglichen Körper (530), der mindestens teilweise flüssigkeitsdurchlässig ist und einen Abschnitt des länglichen Körpers zwischen dem distalen Endbereich und dem proximalen Endbereich aufweist, der nicht von der flüssigkeitsundurchlässigen Barriere abgedeckt ist und positioniert ist, Urin aufzunehmen, der aus einer Harnröhre mindestens in der Nähe des Abschnitts des länglichen Körpers abgeführt wird, wobei der mindestens teilweise flüssigkeitsdurchlässige längliche Körper einschließt:
einen extrudierten Träger (540), der einen distalen Endbereich (548), einen proximalen Endbereich (546) und mindestens einen Schlitz (544a) aufweist, der sich in Längsrichtung auf und entlang des extrudierten Trägers erstreckt, und
eine flüssigkeitsdurchlässige Membran (532), die sich über den mindestens einen Schlitz auf dem extrudierten Träger erstreckt und diesen abdeckt;
wobei der extrudierte Träger einen Kanal (542) einschließt, der sich in Längsrichtung dort hindurch erstreckt und mit dem mindestens einen Schlitz und der Öffnung der flüssigkeitsundurchlässigen Barriere in fluider Verbindung steht;
wobei der distale Endbereich (518) der flüssigkeitsundurchlässigen Barriere eine distale Endkappe (518) einschließt, die mindestens einen darin positionierten Abschnitt des distalen Endbereichs (548) des extrudierten Trägers (540) aufweist, wobei ein Hohlraum-Reservoir (508), zwischen dem distalen Endbereich des extrudierten Trägers und mindestens einem Abschnitt der distalen Endkappe positioniert ist und wirksam ist, eine fluide Verbindung zwischen dem mindestens einen Schlitz und dem Kanal bereitzustellen;
wobei der proximale Endbereich der flüssigkeitsundurchlässigen Barriere eine proximale Endkappe (516) einschließt, die mindestens einen darin positionierten Abschnitt des proximalen Endbereichs (546) des extrudierten Trägers (540) aufweist, und
wobei der extrudierte Träger (540) eine Rückseite und eine Vorderseite einschließt, die zwei seitliche Schlitze (544b) einschließen, die sich in Längsrichtung auf und entlang der Vorderseite des extrudierten Trägers erstrecken, wobei der mindestens eine Schlitz (544a) zwischen den beiden seitlichen Schlitzen positioniert ist; und
wobei jeweilige Abschnitte der flüssigkeitsdurchlässigen Membran innerhalb jedes der beiden seitlichen Schlitze positioniert sind oder sich über die beiden seitlichen Schlitze auf der Vorderseite des extrudierten Trägers erstrecken.

2. Flüssigkeitssammelvorrichtung nach Anspruch 1, wobei:
die flüssigkeitsundurchlässige Barriere eine im Wesentlichen flüssigkeitsundurchlässige Folie einschließt, die mit einem Haftmittel an einem Abschnitt des länglichen Körpers gesichert ist und sich zwischen der proximalen Endkappe und der distalen Endkappe erstreckt.

3. Flüssigkeitssammelvorrichtung nach Anspruch 1 oder 2, wobei ein Abschnitt der flüssigkeitsdurchlässigen Membran innerhalb jedes der beiden seitlichen Schlitze positioniert ist.

4. Flüssigkeitssammelvorrichtung nach Anspruch 3, weiter umfassend ein Formgedächtnispolymer oder -metall (560), das innerhalb jedes der beiden seitlichen Schlitze positioniert ist und den innerhalb der beiden seitlichen Schlitze positionierten Abschnitt der flüssigkeitsdurchlässigen Membran sichert.

5. Flüssigkeitssammelvorrichtung nach Anspruch 4, wobei das Formgedächtnispolymer oder -metall ein längliches U-förmiges Formgedächtnispolymer oder -metall einschließt, das sich durch die distale Endkappe hindurch und in die beiden seitlichen Schlitze hinein erstreckt.

6. Flüssigkeitssammelvorrichtung nach einem der vorstehenden Ansprüche, wobei die flüssigkeitsdurchlässige Membran so um die Vorderseite und die Rückseite des extrudierten Trägers gewickelt ist, dass ein Abschnitt der flüssigkeitsdurchlässigen Membran den mindestens einen Schlitz und die beiden seitlichen Schlitze abdeckt und sich mindestens teilweise über die Öffnung der flüssigkeitsundurchlässigen Barriere erstreckt.

7. Flüssigkeitssammelvorrichtung nach Anspruch 6, weiter umfassend ein Formgedächtnispolymer oder -metall, das sich in Längsrichtung durch die distale Endkappe und mindestens einen Abschnitt des extrudierten Trägers erstreckt.

8. Flüssigkeitssammelvorrichtung nach einem der vorstehenden Ansprüche, wobei die distale Endkappe ein Ausrichtungsmerkmal einschließt, das sich am distalen Endbereich des extrudierten Trägers teilweise in den Kanal hinein erstreckt, und wobei die proximale Endkappe einen inneren Anschluss einschließt, der sich teilweise in den Kanal hinein erstreckt, wobei das Ausrichtungsmerkmal einen oder mehrere Spalte einschließt, die eine fluide Verbindung zwischen dem Reservoir und dem Kanal bereitstellen.

9. Flüssigkeitssammelvorrichtung nach einem der vorstehenden Ansprüche, wobei der extrudierte Träger eine geschlossenzellige Schaumextrusion einschließt.

10. Flüssigkeitssammelvorrichtung nach Anspruch 4 oder einem der Ansprüche 5 bis 9, wenn abhängig von Anspruch 4, wobei die flüssigkeitsdurchlässige Membran (532) innerhalb jedes der beiden seitlichen Schlitze (544b) mindestens eine Teilschleife einschließt und das Formgedächtnispolymer oder -metall (560) ein längliches U-förmiges Formgedächtnispolymer oder -metall einschließt, das sich durch den distalen Endbereich (518) hindurch und in die mindestens eine Teilschleife in jedem der Seitenschlitze (544b) hinein erstreckt.

11. Flüssigkeitssammelvorrichtung nach einem der vorstehenden Ansprüche, wobei die distale Endkappe (518, 618) an ihrer Innenfläche eine oder mehrere Laschen aufweist, die positioniert sind, um zu verhindern oder zu unterdrücken, dass der extrudierte Träger (540, 640) zu weit in die distale Endkappe hineingeschoben wird.

12. Flüssigkeitssammelvorrichtung nach einem der vorstehenden Ansprüche, wobei die Endkappe (518, 618) ein Ausrichtungsmerkmal (519) einschließt, das sich am distalen Endbereich des extrudierten Trägers teilweise in den Kanal (542, 642) hinein erstreckt.

13. Flüssigkeitssammelvorrichtung nach Anspruch 12, wobei das Ausrichtungsmerkmal dazu ausgebildet ist, eine fluide Verbindung zwischen dem Kanal und dem Reservoir bereitzustellen.

14. Flüssigkeitssammelvorrichtung nach Anspruch 12 oder 13, wobei das Ausrichtungsmerkmal eine oder mehrere Laschen oder Anschläge einschließt, um zu verhindern, dass das Ausrichtungsmerkmal zu weit in den Kanal (542, 642) eingeführt wird.

15. Flüssigkeitssammelvorrichtung nach einem der vorstehenden Ansprüche, wobei die distale Kappe (518, 618) Drahtbuchsen für ein U-förmiges Formgedächtnispolymer oder -metall (560) oder eine Buchse (665) für ein verformbares Band (660) einschließt.

## Revendications

1. Dispositif (500) de collecte de fluide, comprenant :
une barrière imperméable aux fluides, présentant une région d'extrémité distale (518) et une région d'extrémité proximale (516) définissant un orifice (512) d'une taille et d'une configuration telles que de l'urine soit aspirée à travers lui par une source de vide ; et
un corps allongé (530), qui est au moins partiellement perméable aux fluides et présente une portion du corps allongé entre la région d'extrémité distale et la région d'extrémité proximale qui n'est pas recouverte par la barrière imperméable aux fluides et positionnée pour recevoir de l'urine évacuée d'un urètre au moins à proximité de la portion du corps allongé, dans lequel le corps allongé qui est au moins partiellement perméable aux fluides inclut :
un support extrudé (540), présentant une région d'extrémité distale (548), une région d'extrémité proximale (546), et au moins une fente (544a) s'étendant longitudinalement sur le support extrudé et selon une direction longitudinale de celui-ci, et
une membrane (532) perméable aux fluides, s'étendant sur l'ensemble de ladite au moins une fente formée sur le support extrudé, et recouvrant cette fente ;
dans lequel le support extrudé inclut un canal (542) s'étendant longitudinalement à travers ce support et étant en communication fluidique avec ladite au moins une fente et avec l'orifice de la barrière imperméable aux fluides ;
dans lequel la région d'extrémité distale (518) de la barrière imperméable aux fluides inclut un capuchon (518) d'extrémité distale présentant au moins une portion de la région d'extrémité distale (548) du support extrudé (540) positionnée en lui, un réservoir (508), constitué par un espace vide, étant positionné entre la région d'extrémité distale du support extrudé et au moins une portion du capuchon d'extrémité distale de manière à assurer une communication fluidique entre ladite au moins une fente et le canal ;
la région d'extrémité proximale de la barrière imperméable aux fluides inclut un capuchon (516) d'extrémité proximale présentant au moins une portion de la région d'extrémité proximale (546) du support extrudé (540) positionnée en lui, et
dans lequel le support extrudé (540) inclut une face arrière et une face avant incluant deux fentes latérales (544b) s'étendant longitudinalement sur la face avant du support extrudé et dans une direction longitudinale de cette face, ladite au moins une fente (544a) étant positionnée entre les deux fentes latérales ; et
des portions respectives de la membrane perméable aux fluides sont positionnées dans chacune des deux fentes latérales ou s'étendent sur l'ensemble des deux fentes latérales sur la face avant du support extrudé.

2. Dispositif de collecte de fluide selon la revendication 1, dans lequel
la barrière imperméable aux fluides inclut une feuille sensiblement imperméable aux fluides, ladite feuille étant fixée à une portion du corps allongé avec un adhésif et s'étendant entre le capuchon d'extrémité proximale et le capuchon d'extrémité distale.

3. Dispositif de collecte de fluide selon la revendication 1 ou la revendication 2, dans lequel une portion de la membrane perméable aux fluides est positionnée dans chacune des deux fentes latérales.

4. Dispositif de collecte de fluide selon la revendication 3, comprenant en outre un polymère ou métal à mémoire de forme (560) positionné dans chacune des deux fentes latérales et fixant la portion de la membrane perméable aux fluides qui est positionnée dans les deux fentes latérales.

5. Dispositif de collecte de fluide selon la revendication 4, dans lequel le polymère ou métal à mémoire de forme inclut un polymère ou métal à mémoire de forme allongé en forme de U s'étendant à travers le capuchon d'extrémité distale et dans les deux fentes latérales.

6. Dispositif de collecte de fluide selon l'une quelconque des revendications précédentes, dans lequel la membrane perméable aux fluides est enroulée autour de la face avant et de la face arrière du support extrudé de telle sorte qu'une portion de la membrane perméable aux fluides recouvre ladite au moins une fente et les deux fentes latérales, et de telle sorte qu'elle s'étend au moins partiellement sur l'ensemble de l'ouverture de la barrière imperméable aux fluides.

7. Dispositif de collecte de fluide selon la revendication 6, comprenant en outre un polymère ou métal à mémoire de forme s'étendant longitudinalement à travers le capuchon d'extrémité distale et au moins une partie du support extrudé.

8. Dispositif de collecte de fluide selon l'une quelconque des revendications précédentes, dans lequel le capuchon d'extrémité distale inclut un élément d'alignement s'étendant partiellement dans le canal au niveau de la région d'extrémité distale du support extrudé et le capuchon d'extrémité proximale inclut un orifice interne s'étendant partiellement dans le canal, l'élément d'alignement incluant une ou plusieurs fentes assurant une communication fluidique entre le réservoir et le canal.

9. Dispositif de collecte de fluide selon l'une quelconque des revendications précédentes, dans lequel le support extrudé inclut une extrusion de mousse à cellules fermées.

10. Dispositif de collecte de fluide selon la revendication 4 ou l'une quelconque des revendications 5 à 9 en tant qu'elle est rattachée à la revendication 4, dans lequel la membrane (532) perméable aux fluides inclut au moins une boucle partielle dans chacune des deux fentes latérales (544b) et le polymère ou métal à mémoire de forme (560) inclut un polymère ou métal à mémoire de forme allongé en forme de U s'étendant à travers la région d'extrémité distale (518) et dans ladite au moins une boucle partielle dans chacune des fentes latérales (544b).

11. Dispositif de collecte de fluide selon l'une quelconque des revendications précédentes, dans lequel le capuchon d'extrémité distale (518, 618) présente une ou plusieurs languettes sur sa surface interne positionnées de manière à empêcher que le support extrudé (540, 640) soit poussé trop loin dans le capuchon d'extrémité distale ou pour inhiber une telle poussée.

12. Dispositif de collecte de fluide selon l'une quelconque des revendications précédentes, dans lequel le capuchon d'extrémité (518, 618) inclut un élément d'alignement (519) s'étendant partiellement dans le canal (542, 642) au niveau de la région d'extrémité distale du support extrudé.

13. Dispositif de collecte de fluide selon la revendication 12, dans lequel l'élément d'alignement est tel qu'il réalise une communication fluidique entre le canal et le réservoir.

14. Dispositif de collecte de fluide selon la revendication 12 ou la revendication 13, dans lequel l'élément d'alignement inclut une ou plusieurs languettes ou butées pour empêcher l'élément d'alignement d'être inséré trop loin dans le canal (542, 642).

15. Dispositif de collecte de fluide selon l'une quelconque des revendications précédentes, dans lequel le capuchon distal (518, 618) inclut des logements pour fil pour un fil en forme de U en polymère ou métal à mémoire de forme (560), ou un logement (665) pour une bande malléable (660).
